# EUROPÄISCHE PATENTANMELDUNG

(11) **EP 2 347 738 A1**
(43) Veröffentlichungstag der Anmeldung: **27.07.2011**
(21) Anmeldenummer: 11151558.1
(22) Anmeldetag: 20.01.2011
(51) Int. Cl.: A61F 5/02

(54) **Körper-Stützgerät**

(30) Priorität: 21.01.2010 DE 102010000145
(71) Anmelder: Kolmeder, Georg, 84048 Mainburg (DE)
(72) Erfinder: Kolmeder, Georg, 84048 Mainburg (DE)
(74) Vertreter: Gustorf, Gerhard

(57) **Zusammenfassung**

2. Das Stützgerät dient zum Entlasten der Wirbelsäule sowie der Hüft- und Kniegelenke von in gebückter und/oder kniender Haltung arbeitenden Personen und hat einen Brustschild (22), an dem Stützorgane zur Aufnahme der Gewichtskraft der Person angelenkt sind. Die Stützorgane bestehen aus zwei nebeneinander angeordneten, i.w. vertikalen Stangen (16), deren untere Enden zur Aufnahme der abzustützenden Gewichtskraft ausgebildet sind.

## Beschreibung

Die Erfindung betrifft ein Stützgerät zum Entlasten der Wirbelsäule sowie der Hüft- und Kniegelenke von in gebückter und/oder kniender Haltung arbeitenden Personen mit einem Brustschild, an dem Stützorgane zur Aufnahme der Gewichtskraft der Person angelenkt sind.

Gegenstand des Gebrauchsmusters 297 05 124 ist ein Stützgerät für Personen beim Arbeiten in gebückter Haltung. Dieses besteht aus einem Brustbügel mit einem waagrechten Stützriegel, von dessen beiden Enden je ein Federarm nach unten absteht, dessen unteres Ende als waagrechter Oberschenkelbügel ausgebildet ist. Dieses einteilige Stützgerät kann innerhalb sehr kurzer Zeit leicht angelegt werden und entlastet insbesondere die Wirbelsäule bei Arbeiten in gebückter Haltung, wobei die Gewichtskraft über den Federarm auf den Oberschenkel übertragen wird.

In dem Gebrauchsmuster 20 2007 008 409 ist ein Wirbelsäulen-Stützgerät der eingangs umrissenen Bauart beschrieben und dargestellt, das mehrteilig ausgebildet ist und einen geschlossenen Rahmen hat, an dem über ein Drehgelenk auf jeder Seite ein Horizontalarm schwenkbar befestigt ist, dessen freie Enden, die über federnde Zugelemente mit dem Rahmen verbunden sind, ebenfalls als Oberschenkelbügel ausgebildet sind, die die Gewichtskraft der Person am Oberschenkel abstützen. Dieses Gerät eignet sich vorwiegend für Arbeiten in kniender Stellung, beispielsweise für Fliesenleger, Estrichleger oder dgl.

Der Erfindung liegt die Aufgabe zugrunde, ein weiterentwickeltes Stützgerät zur Verfügung zu stellen, das bei vereinfachtem Aufbau eine Entlastung der Wirbelsäule sowie der Hüft- und Kniegelenke sowohl in kniender als auch in gebückter Stellung bewirkt und dabei auch die jeweils betroffene Muskulatur entlastet.

Diese Aufgabe wird gemäß der Erfindung dadurch gelöst, dass die Stützorgane aus zwei nebeneinander angeordneten, im wesentlichen vertikalen Stangen bestehen, deren untere Enden zur Aufnahme der abzustützenden Gewichtskraft ausgebildet sind. Dabei ist es von Vorteil, wenn die Stangen von jeweils einer Führungshülse in Richtung ihrer Längsachse verschiebbar aufgenommen werden.

Das Stützgerät gemäß der Erfindung stellt gegenüber dem Stand der Technik eine wesentliche Vereinfachung dar, weil alle Gewichtskräfte, die von der arbeitenden Person ausgeübt werden, unmittelbar über die vertikalen Stangen nach unten, vorzugsweise auf den Boden geleitet werden.

In kniender Arbeitsstellung mit nach vom geneigter Haltung, z.B. eines Estrich-, Fliesen- oder Bodenlegers wird die Gewichtskraft des Oberkörpers über die beiden vertikalen Stangen vollständig auf den Boden übertragen. Hierdurch ergibt sich eine nahezu völlige Entlastung der Wirbelsäule und des Hüftgelenks, wobei gleichzeitig auch das Kniegelenk um 30% bis 60% entlastet wird, weil es das Gewicht des Oberkörpers, das im Mittel bei 40 kg liegt, nicht mehr aufnehmen muss.

Das Stützgerät gemäß der Erfindung eignet sich daneben auch für stehende Tätigkeiten mit nach vom gebeugtem Oberkörper, beispielsweise für Monteure an einem Fließband. Hierbei wird die Gewichtskraft des Oberkörpers dadurch auf die Oberschenkel übertragen, dass die Stangen innerhalb der Führungshülsen, die an der Arbeitshose oder an einem Knieschoner befestigt werden, durch Feststellelemente blockiert werden können. Alternativ können die unteren Enden der beiden Stangen mittels eines Schnellverschlusselementes an jeweils einem Oberschenkelgurt angelenkt sein; in diesem Falle sind keine Führungshülsen erforderlich.

Weitere Merkmale der Erfindung ergeben sich aus den Patentansprüchen.

Die Erfindung ist nachstehend an Ausführungsbeispielen erläutert, die in der Zeichnung dargestellt sind. Es zeigen:
Figur 1 die Vorderansicht eines Stützgerätes gemäß der Erfindung im angelegten Zustand,
Figur 2 eine Seitenansicht der in Figur 1 gezeigten Person in kniender Haltung,
Figur 3 eine Frontansicht der in Figur 2 gezeigten Person,
Figur 4 die schematische Darstellung einer vertikalen Stange in ihrer Führungshülse,
Figur 5 eine Variante der Figur 4,
Figur 6 die Seitenansicht einer an der Innenseite eines Knieschoners oder einer Arbeitshose befestigten Führungshülse,
Figur 7 das Stützgerät gemäß der Erfindung bei Arbeiten in gebückter Haltung und
Figur 8 eine Variante der Figur 7.

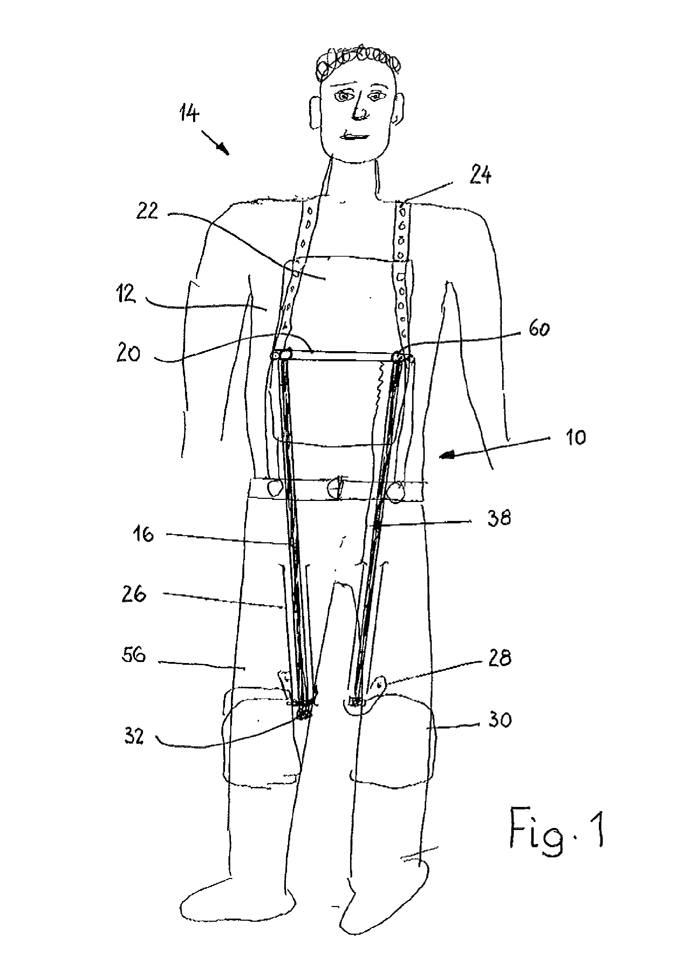

Wie Figur 1 zeigt, hat das Stützgerät 10 gemäß der Erfindung als Stützorgan zur Aufnahme der vom Oberkörper 12 einer Person 14 ausgeübten Gewichtskraft zwei im wesentlichen vertikale Stangen 16, die aus Metall oder einem festen Kunststoff hergestellt sind.

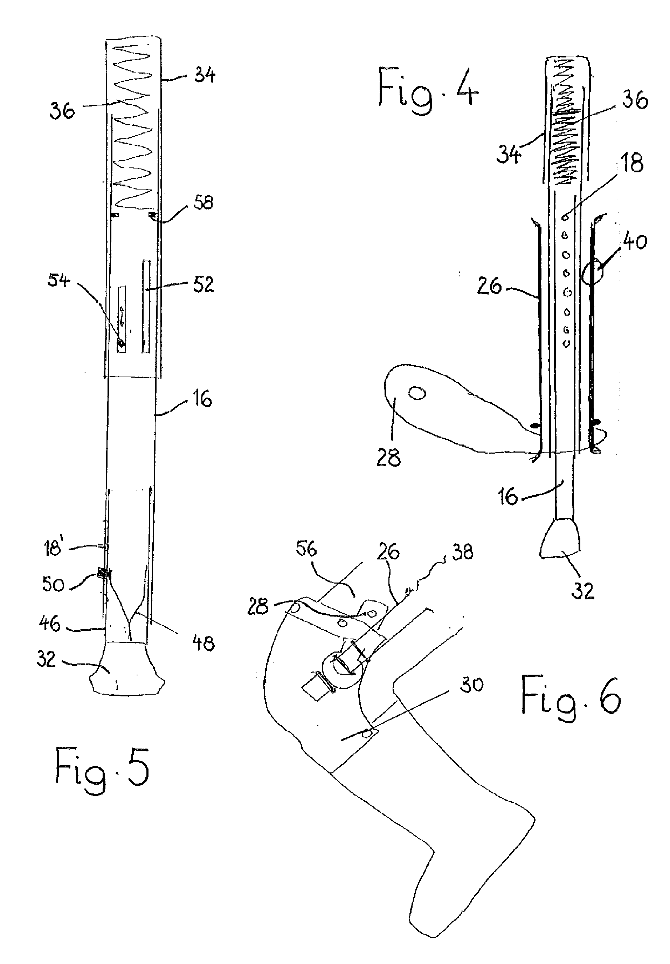

In Figur 4 und 5 ist angedeutet, dass die Stangen 16 teleskopisch längenverstellbar sind und zur Fixierung der eingestellten Länge entsprechende Rastlöcher 18, 18' haben.

Aus Figur 1 geht weiter hervor, dass die oberen Enden der beiden Stangen 16 über Fixierelemente 60 horizontal verstellbar an einem Querstab 20 befestigt sind, der höhenverstellbar an einem Brustschild 22 angebracht ist. Der Brustschild 22 ist auf seiner zum Oberkörper 12 weisenden Seite gepolstert und zwischen zwei Hosenträgern 24 nach oben und unten verstellbar befestigt, beispielsweise durch Knöpfe.

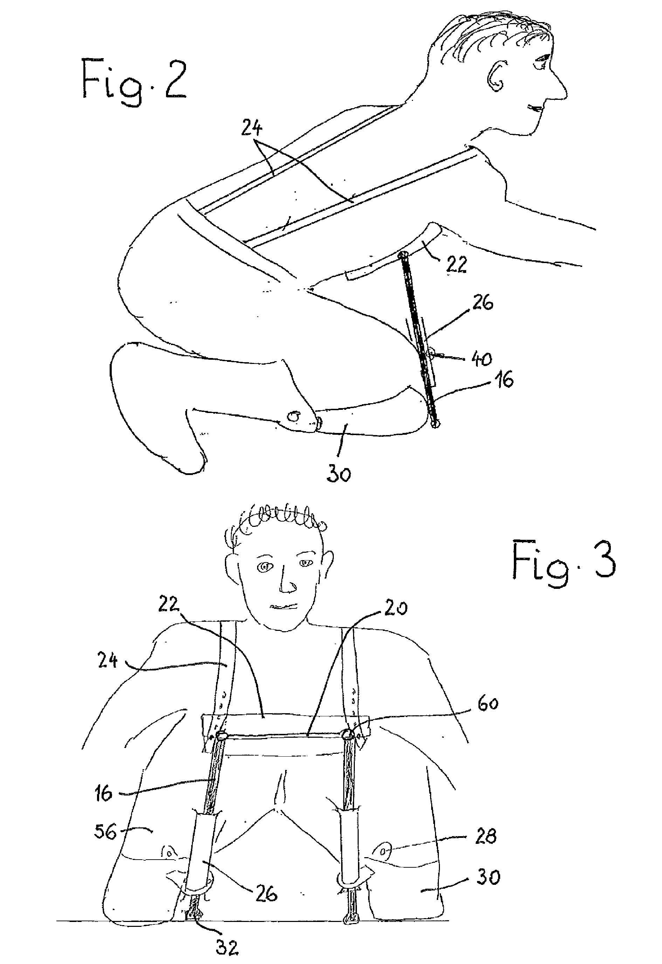

Jede der beiden Stangen 16 ist in einer Führungshülse 26 in Richtung ihrer Längsachse verschiebbar aufgenommen. In den Figuren 3, 4 und 6 ist angedeutet, dass das untere Ende jeder Führungshülse 26 eine Lasche 28 trägt, die an der Innenseite eines Knieschoners 30 oder einer Arbeitshose 56 befestigt werden kann. Die an der Knieinnenseite der Arbeitshose 56 fixierte Lasche 28 sorgt dafür, dass der untere Abstützpunkt (Pufferelement 32) immer an der gleich bleibenden, ergonomisch günstigsten Stelle liegt.

Die Figuren 4 und 5 zeigen weiter, dass in vorteilhafter Weise am unteren Ende jeder Stange 16 ein Pufferelement 32 befestigt ist, das aus elastischem Material besteht und die Aufgabe hat, bei Arbeiten in kniender Stellung (Figuren 2 und 3) eine rutschfeste Auflage auf dem Boden zu bilden und gleichzeitig diesen vor Verkratzungen zu schützen.

Figur 4 zeigt ferner, dass jede Stange 16 an ihrem oberen Ende eine Stoßdämpferhülse 34 hat, die über eine Druckfeder 36 am oberen Ende der Stange 16 abgestützt ist. Alternativ kann statt der Druckfeder 36 auch ein anderes Federelement vorgesehen sein, beispielsweise ein Gasdruckdämpfer. Die Stoßdämpferhülse 34 vermeidet bei Arbeiten in gebückter Haltung mit Bewegungen des Oberkörpers 12 nach unten harte Stöße gegen das Brustschild 22 und damit auch gegen den Oberkörper 12. Durch die Federwirkung wird auch das Aufrichten des Körpers aus der gebückten Haltung unterstützt und erleichtert.

In der Ausführungsform der Figur 5 ist das Pufferelement 32 am unteren Ende eines rohrförmigen Fußes 46 angebracht, der teleskopisch verstellbar in der Stange 16 aufgenommen ist. Diese hat an einer Seite eine Reihe von Rastlöchern 18', in die gegen eine Blattfeder 48 oder dergleichen verschiebbarer Druckknopf 50 einrasten kann, um die jeweils gewünschte Länge zu fixieren.

Weiterhin zeigt Figur 5 die Möglichkeit, den Federweg der Druckfeder 36 einzustellen, die sich mit ihrem unteren Ende an einem Anschlag 58 in der hohlen Stange 16 abstützt. Hierzu sind in die verdrehbare Stoßdämpferhülse 34 über den Umfang verteilt mehrere Schlitze 52 verschiedener Länge eingearbeitet, in die wahlweise ein gegen eine Federkraft eindrückbarer Stellknopf 54 eingreift, der an der Stange 16 angebracht ist.

In Figur 1 ist dargestellt, dass die oberen Enden der beiden Führungshülsen 26 über ein elastisches Band 38 an dem Querstab 20 aufgehängt sind, um ein Auseinanderfallen des Stützgerätes 10 beim Anlegen oder Ablegen zu vermeiden.

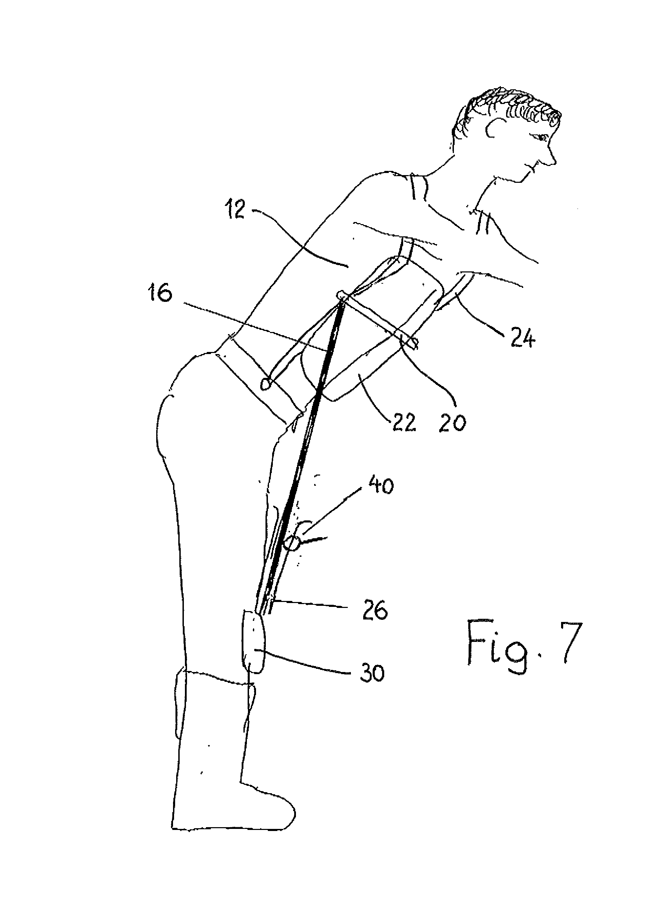

In den Figuren 4 und 7 ist angedeutet, dass jede Führungshülse 26 ein Feststellelement 40 haben kann, beispielsweise eine Klemmschraube, um die Stange 16 gegen Verschieben in der Führungshülse 26 zu blockieren. Dies ist beispielsweise im Fall von Figur 7 von Vorteil, wenn das Stützgerät 10 in gebückter Haltung der Person 14 verwendet wird. In diesem Fall ist, wie Figur 7 zeigt, die Führungshülse 26 so an einem Knieschoner 30 oder der Arbeitshose 56 befestigt, beispielsweise mittels einer in Figur 4 gezeigten Lasche 28, dass das Gewicht des Oberkörpers 12 an dieser Stelle abgestützt ist. Wenn die Lasche 28 aus Gummi oder einem anderen elastischen Material besteht, wird zusätzlich eine Federwirkung erzielt, die bei durch das Feststellelement 40 blockiertem Stützgerät 10 eine größere Bewegungsfreiheit gewährt.

Wenn keine Bewegungsfreiheit beim Vorbeugen des Oberkörpers 10 gewünscht ist (Figur 7), sollte die Lasche 28 aus einem steifen Material bestehen, um ein in sich starres System zu schaffen.

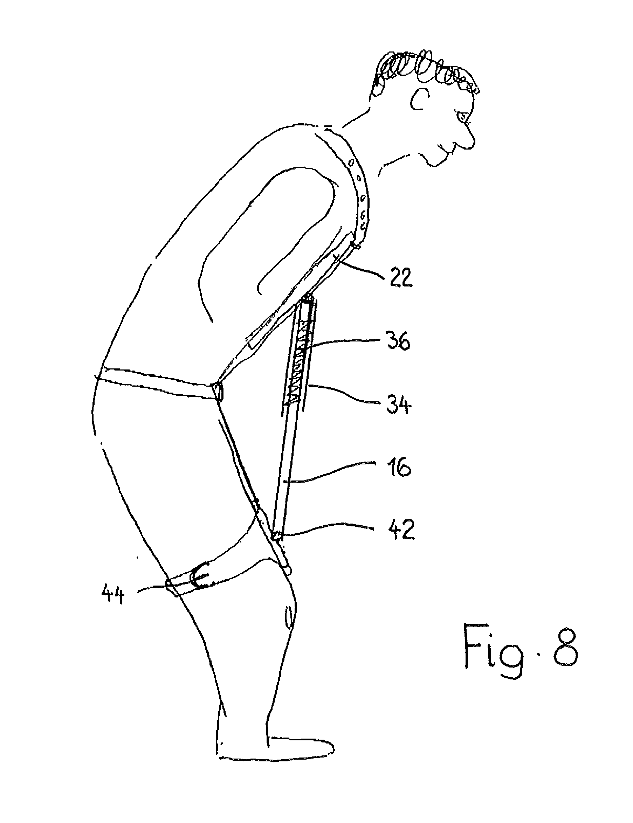

Figur 8 zeigt eine Alternative zu Figur 7, bei der das untere Ende jeder Stange 16 mittels eines Schnellverschlusselementes 42, beispielsweise eines Klettbandes, an einem gepolsterten Oberschenkelgurt 44 angelenkt ist. In diesem Fall sind die Führungshülsen 26 der Figur 7 entbehrlich.

## Patentansprüche

1. Stützgerät zum Entlasten der Wirbelsäule sowie der Hüft- und Kniegelenke von in gebückter und/oder kniender Haltung arbeitenden Personen mit einem Brustschild (22), an dem Stützorgane zur Aufnahme der Gewichtskraft der Person angelenkt sind, **dadurch gekennzeichnet, dass** die Stützorgane aus zwei nebeneinander angeordneten, i.w. vertikalen Stangen (16) bestehen, deren untere Enden zur Aufnahme der abzustützenden Gewichtskraft ausgebildet sind.

2. Stützgerät nach Anspruch 1, **dadurch gekennzeichnet, dass** die Stangen (16) von jeweils einer Führungshülse (26) in Richtung ihrer Längsachse verschiebbar aufgenommen sind.

3. Stützgerät nach Anspruch 1 oder 2, **dadurch gekennzeichnet, dass** die Stangen (16) teleskopisch längenverstellbar sind.

4. Stützgerät nach einem der vorhergehenden Ansprüche, **dadurch gekennzeichnet, dass** an dem unteren Ende jeder Stange (16) ein Pufferelement (32) angebracht ist.

5. Stützgerät nach einem der vorhergehenden Ansprüche, **dadurch gekennzeichnet, dass** die Stangen (16) an ihrem oberen Ende jeweils eine gegen eine Federkraft verschiebbare Stoßdämpferhülse (34) haben.

6. Stützgerät nach Anspruch 5, **dadurch gekennzeichnet, dass** die Federkraft und/oder der Federweg der Feder (36) einstellbar ist.

7. Stützgerät nach einem der vorhergehenden Ansprüche, **dadurch gekennzeichnet, dass** die oberen Enden der beiden Stangen (16) horizontal verstellbar an einem Querstab (20) befestigt sind, der an dem Brustschild (22) höhenverstellbar angebracht ist.

8. Stützgerät nach Anspruch 7, **dadurch gekennzeichnet, dass** der Brustschild (22) Mittel zur höhenverstellbaren, lösbaren Befestigung an Hosenträgern (24) hat.

9. Stützgerät nach einem der vorhergehenden Ansprüche, **dadurch gekennzeichnet, dass** jede Führungshülse (26) ein Feststellelement (40) zum Blockieren der Verschiebbarkeit der Stange (16) in der Führungshülse (26) hat.

10. Stützgerät nach einem der vorhergehenden Ansprüche, **dadurch gekennzeichnet, dass** an dem unteren Ende jeder Führungshülse (26) Mittel (28) zur Befestigung an einem Knieschoner (30), einem Arbeitsschuh oder einer Arbeitshose (56) vorgesehen sind.

11. Stützgerät nach Anspruch 10, **dadurch gekennzeichnet, dass** die Mittel zur Befestigung aus einer Lasche (28) aus elastischem oder steifem Material bestehen.

12. Stützgerät nach einem der Ansprüche 1 bis 9, **dadurch gekennzeichnet, dass** die unteren Enden der beiden Stangen (16) mittels eines Schnellverschlusselementes (42) an jeweils einem Oberschenkelgurt (44) angelenkt sind.
